# EUROPEAN PATENT APPLICATION

(11) **EP 1 365 024 A1**
(43) Date of publication of application: **26.11.2003**
(21) Application number: 02701634.4
(22) Date of filing: 28.02.2002
(51) Int. Cl.: C12N 15/12, C12N 1/19, C12N 1/21, C12N 5/10, C07K 14/705, C07K 16/28, C12P 21/02, C12Q 1/68, A61K 45/00, A61P 1/00, A61P 9/00, A61P 11/00, A61P 17/00, A61P 19/00, A61P 25/00, A61P 29/00, A61P 31/00, A61P 35/00, A61P 37/00

(54) **TRAF3-BINDING B CELL-SPECIFIC RECEPTOR**

(30) Priority: 28.02.2001 JP 2001055119
(71) Applicant: Riken, Wako-shi, Saitama 351-0198 (JP); Irie, Shinji, Ichihara-shi, Chiba 299-0114 (JP); Sato, Takaaki, Fort Lee, NJ 07024 (US)
(72) Inventor: IRIE, Shinji, Ichihara-shi, Chiba 299-0114 (JP); SATO, Takaaki, Fort Lee, NJ 07024 (US)
(74) Representative: Arends, William Gerrit
(86) International application number: JP0201849
(87) International publication number: WO02072827

(57) **Abstract**

To identify a signal transduction molecule which binds to TRAF3. A protein having any of the following amino acid sequences:
(a) an amino acid sequence of SEQ ID NO: 1;
(b) an amino acid sequence obtained by deletion, substitution, and/or insertion of one or several of the amino acids in the amino acid sequence of SEQ ID NO: 1 and having activity of binding to TRAF3; or
(c) an amino acid sequence having a homology of 60% or more to the amino acid sequence of SEQ ID NO: 1 and having activity of binding to TRAF3.

SEQUENCE LISTING

<110> RIKEN

<120> A TRAF3-binding B cell specific receptor

<130> A21041M

<160> 2

<210> 1

<211> 175

<212> PRT

<213> Mouse

<400> 1

Met Gly Ala Arg Arg Leu Arg Phe Arg Ser Gln Arg Ser Arg Asp Ser
1               5                   10                  15

Ser Val Pro Thr Gln Cys Asn Gln Thr Glu Cys Phe Asp Pro Leu Val
            20                  25                  30

Arg Asn Cys Val Ser Cys Glu Leu Phe His Thr Pro Asp Thr Gly His
        35                  40                  45

Thr Ser Ser Leu Glu Pro Gly Thr Ala Leu Gln Pro Gln Glu Gly Ser
    50                  55                  60

Ala Leu Arg Pro Asp Val Ala Leu Leu Val Gly Ala Pro Ala Leu Leu
65                  70                  75                  80

Gly Leu Ile Leu Ala Leu Thr Leu Val Gly Leu Val Ser Leu Val Ser
                85                  90                  95

Trp Arg Trp Arg Gln Gln Leu Arg Thr Ala Ser Pro Asp Thr Ser Glu
            100                 105                 110

Gly Val Gln Gln Glu Ser Leu Glu Asn Val Phe Val Pro Ser Ser Glu

1/3

115 120 125
Thr Pro His Ala Ser Ala Pro Thr Trp Pro Pro Leu Lys Glu Asp Ala
130 135 140
Asp Ser Ala Leu Pro Arg His Ser Val Pro Val Pro Ala Thr Glu Leu
145 150 155 160
Gly Ser Thr Glu Leu Val Thr Thr Lys Thr Ala Gly Pro Glu Gln
165 170 175

<210> 2
<211> 528
<212> DNA
<213> Mouse
<400> 2
ATG GGC GCC AGG AGA CTC CGG TTC CGA AGC CAG AGG AGC CGG GAC AGC 48
Met Gly Ala Arg Arg Leu Arg Phe Arg Ser Gln Arg Ser Arg Asp Ser
1               5                   10                  15
TCG GTG CCC ACC CAG TGC AAT CAG ACC GAG TGC TTC GAC CCT CTG GTG 96
Ser Val Pro Thr Gln Cys Asn Gln Thr Glu Cys Phe Asp Pro Leu Val
            20                  25                  30
AGA AAC TGC GTG TCC TGT GAG CTC TTC CAC ACG CCG GAC ACT GGA CAT 144
Arg Asn Cys Val Ser Cys Glu Leu Phe His Thr Pro Asp Thr Gly His
        35                  40                  45
ACA AGC AGC CTG GAG CCT GGG ACA GCT CTG CAG CCT CAG GAG GGC TCC 192
Thr Ser Ser Leu Glu Pro Gly Thr Ala Leu Gln Pro Gln Glu Gly Ser
    50                  55                  60
GCG CTG AGA CCC GAC GTG GCG CTG CTC GTC GGT GCC CCC GCA CTC CTG 240
Ala Leu Arg Pro Asp Val Ala Leu Leu Val Gly Ala Pro Ala Leu Leu

65                    70                    75                    80

GGA CTG ATA CTG GCG CTG ACC CTG GTG GGT CTA GTG AGT CTG GTG AGC  288

Gly Leu Ile Leu Ala Leu Thr Leu Val Gly Leu Val Ser Leu Val Ser

                    85                    90                    95

TGG AGG TGG CGT CAA CAG CTC AGG ACG GCC TCC CCA GAC ACT TCA GAA  336

Trp Arg Trp Arg Gln Gln Leu Arg Thr Ala Ser Pro Asp Thr Ser Glu

                100                   105                   110

GGA GTC CAG CAA GAG TCC CTG GAA AAT GTC TTT GTA CCC TCC TCA GAA  384

Gly Val Gln Gln Glu Ser Leu Glu Asn Val Phe Val Pro Ser Ser Glu

            115                   120                   125

ACC CCT CAT GCC TCA GCT CCT ACC TGG CCT CCG CTC AAA GAA GAT GCA  432

Thr Pro His Ala Ser Ala Pro Thr Trp Pro Pro Leu Lys Glu Asp Ala

        130                   135                   140

GAC AGC GCC CTG CCA CGC CAC AGC GTC CCG GTG CCC GCC ACA GAA CTG  480

Asp Ser Ala Leu Pro Arg His Ser Val Pro Val Pro Ala Thr Glu Leu

145                   150                   155                   160

GGC TCC ACC GAG CTG GTG ACC ACC AAG ACA GCT GGC CCA GAG CAA TAG

Gly Ser Thr Glu Leu Val Thr Thr Lys Thr Ala Gly Pro Glu Gln

                    165                   170                   175

## Description

### Technical Field

The present invention relates to a novel TRAF3-binding protein which is a members of B-cell-specific receptors. The present invention also relates to a gene encoding said protein, a vector having said gene, a transformant having said gene, an antibody which recognizes said protein, a process for screening medicines by utilizing said protein, and a process for diagnosis by utilizing said protein.

### Background Art

Biological mechanisms are controlled by a response to certain stimulus. Specifically, many cytokines bind to receptors and induce intracellular reactions. For example, tumor necrosis factor (TNF) functions via TNF receptor and controls biological processes such as induction of infection and shock or protection against inflammatory diseases. TNF belongs to TNF-ligand superfamily and functions together with TNF receptor superfamily which is a ligand thereof. Examples of known ligands include TNF-a, lymphotoxin (LT)- α, LT- β, FasL, CD40L, CD27L, CD30L, OX40L, and nerve growth factors (NGF). Examples of known TNF receptor superfamilies are p55TNF receptor, p75TNF receptor, TNF receptor-associated factors, Fas antigen, CD40, CD27, CD30, OX40, and low-affinity p75 and NGF receptors. Functions of several types of TNF receptor families have already been revealed. For example, Fas antigen, low-affinity NGF receptor, and ligands thereof are involved in programmed cell death, and CD40 ligand is involved in T-cell-dependent B-cell activation.

Programmed cell death which is one of the biological processes is essential for normal growth and homeostasis in a multicellular organism. Deranged apoptosis is related to causes of human diseases including cancer, neurodegenerative diseases, and acquired immunodeficiency syndrome (AIDS). In the activation of Fas, FADD/MORT1 which is a death domain-containing adaptor molecule, binds to an intracellular domain of Fas. The resultant then activates caspase-8, which is proapoptosis protease, and apoptosis is finally carried out. On the other hand, TNFR-1 transmits signals of a series of extensive biological activities mainly by activating NF-κ B. TNFR-1 binds to death domain-containing TRADD in an intracellular domain of TNFR-1, and aggregates with many signal transmitting molecules such as FADD, TRAF2 and RIP, thereby transmitting both signals for apoptosis and NF- κ B activation. Thus, the functions of TNF family ligand and TNF family receptor are diversified, and they affect many biological processes. Therefore, the identification and analysis of these receptors, ligands and signal transmitting molecules associated therewith are important for establishing therapeutic methods for diseases.

TNF receptor-associated factors (TRAF) are a family of proteins that were discovered initially as downstream signal transducers of the TNF receptor superfamily [Rothe,M. et al, (1994) Cell 78, 681-692; Hu, H.M. et al, (1994) J.Biol.Chem. 269, 30069-30072;and Cheng, G. et al, (1995) Science 267, 1494-1498]. To date, six members of the TRAF family have been identified [Rothe,M. et al., (1994) Cell 78, 681-692; Hu, H.M. et al., (1994) J.Biol.Chem. 269, 30069-30072; Cheng, G., et al., (1995) Science 267, 1494-1498; Cao, Z., et al., (1996) Nature (London) 383, 443-446; Mosialos, G, et al., (1995) Cell 80, 389-399; Sato, T., et al., (1995) FEBS Lett. 358, 113-118; Regnier,C.H. et al., (1995) J.Biol.Chem. 270, 25715-25721; Ishida,T.K. et al. (1996) Proc.Natl.Acad.Sci.USA 93, 9437-9442; Nakano, H. et al., (1996) J.Biol.Chem. 271, 14661-14664; and Ishida,T.K. et al., (1996) J.Biol.Chem. 271, 28745-28748]. TRAFs 1-6 are characterized by a coiled-coil TRAF-N domain and a conserved C-terminal TRAF-C domain [Cao, Z. et al., (1996) Nature (London) 383, 443-446]. The TRAF-C domain mediates interactions among TRAF proteins as well as their association with members of the TNFR superfamily [Cheng, G et al., (1995) Science 267, 1494-1498; Takeuchi, M. et al., (1996) J.Biol.Chem. 271, 19935-19942;and Hsu, H. et al., (1996) Cell 84, 299-308].

TRAF3 was originally identified as a molecule that bound to the cytosolic domain of the B cell surface marker, CD40, and the Epstein-Barr virus latent membrane protein, LMP-1 [Mosialos, G et al, (1995) Cell 80, 389-399; Sato, T. et al, (1995) FEBS Lett. 358, 113-118; Hu, H.M. et al, (1994) J.Biol.Chem. 269, 30069-30072; and Cheng, G et al, (1995) Science 267, 1494-1498]. Subsequently, TRAF3 has been shown to interact with cytosolic tail of other TNFR family members, such as lymphotoxin β-receptor [Force, W.R., et al, (1997) J.Biol.Chem. 272, 30835-30840]. Because TRAF family proteins including TRAF3 are cytosolic proteins that appear to lack catalytic activity, it is generally believed to play as the adapter proteins. In this regard, TRAF3 has been found to interact with other signaling molecule such as NIK, ASK and TANK [Song, H.Y. et al, (1997) Proc.Natl.Acad.Sci.USA 94, 9792-9796; Nishitoh, H. et al, (1995) Mol.Cell 2, 389-395; and Regnier,C.H. et al, (1997) Cell 90, 373-383]. However, the mechanism by which TRAF3 mediates signal transduction are not completely understand.

### Disclosure of the Invention

An object of the present invention is to identify a signal-transmitting molecule that binds to TRAF3. Another object of the present invention is to clone a gene encoding a signal-transmitting molecule that binds to TRAF3 and reveal the structure and function of this gene. Further object of the present invention is to provide a novel medicine or a diagnostic process by utilizing a gene encoding a signal-transmitting molecule that binds to TRAF3.

In order to attain the above objects, the present inventors have performed a protein-protein interaction screening utilizing yeast. In this screening, a human-derived Sos (hSos) protein is recruited on a cell membrane to activate the yeast RAS signaling pathway, and yeast cdc25 gene deficiency is complemented. The present inventors have identified the novel TRAF3 binding protein (hereinafter designated T3BP) by this approach with a WEHI231 cell cDNA expression library and full-length TRAF3 fused to hSos, and have revealed the structure and function of this T3BP protein. The present invention have been completed based on these findings.

Thus, the present invention provides a protein having any of the following amino acid sequences:
(a) an amino acid sequence of SEQ ID NO: 1;
(b) an amino acid sequence obtained by deletion, substitution, and/or insertion of one or several of the amino acids in the amino acid sequence of SEQ ID NO: 1 and having activity of binding to TRAF3; or
(c) an amino acid sequence having a homology of 60% or more to the amino acid sequence of SEQ ID NO: 1 and having activity of binding to TRAF3.

The present invention further provides a gene encoding the protein of the present invention.

The present invention further provides a gene having any of the following nucleotide sequences:
(a) a nucleotide sequence of SEQ ID NO: 2;
(b) a nucleotide sequence obtained by deletion, addition, or substitution of one or several of the nucleotides in the nucleotide sequence of SEQ ID NO: 2 and encoding a protein having activity of binding to TRAF3; or
(c) a nucleotide sequence which hybridizes with the nucleotide sequence of SEQ ID NO: 2 under stringent conditions and encoding a protein having activity of binding to TRAF3.

The present invention further provides a vector comprising the gene of the present invention.

The present invention further provides a transformant having the gene or vector of the present invention.

The present invention further provides a process for producing a protein having activity of binding to TRAF3, wherein the transformant of the present invention is used.

The present invention further provides an antibody which recognizes the protein of the present invention.

The present invention further provides a process for screening for a substance that promotes or suppresses functions of the protein or antibody, wherein the protein or antibody of the present invention is used. Preferably, a substance that promotes or suppresses functions of the protein or antibody of the present invention promotes or suppresses intracellular signal transduction via TRAF3.

The present invention further provides a substance that promotes or suppresses functions of the protein of the present invention, which is obtained by the screening process according to the present invention.

The present invention further provides a medicine comprising, as an active ingredient, the aforementioned substance. The medicine according to the present invention is preferably used for preventing or treating diseases associated with abnormalities in intracellular signal transduction via TRAF3.

The present invention further provides a diagnostic process for diseases associated with abnormalities in intracellular signal transduction via TRAF3, which comprises measuring the level of the protein of the present invention in an organism-derived ingredient.

### Brief Description of the Invention

Fig. 1A shows a predicted amino acid sequence of T3BP, and Fig. 1B shows Northern blot analysis of T3BP mRNA.
Fig. 2A shows the analysis results of GST pull down assay which shows the binding property of GST fusion proteins (GST-clone 3, GST-clone 1) to TRAF2 or TRAF3.
Fig. 2B shows the analysis results of GST pull down assay which shows the binding property of GST-T3BP to TRAF2, TRAF3, TRAF5 or TRAF6.
Fig. 2C shows the analysis results of immunoprecipitation assay which shows interaction of TRAF3 and T3BP.
Fig. 3A and 3B shows the results of mapping of T3BP binding region in TRAF3.
Fig. 3C shows a schematic presentation of primary structure of murine TRAF3.
Fig. 4A shows the analysis results of in vitro assay which shows the binding property of GST fusion T3BP truncation mutant to TRAF3.
Fig. 4B shows the analysis results of GST pull down assay which shows the binding property of GST fusion T3BP truncation mutant to TRAF3.
Fig. 4C shows the primary structure of T3BP and its TRAF3 binding region.
Fig. 5A shows the morphological change of cells by expression of T3BP.
Fig. 5B shows the effect of T3BP expression on the cellular distribution of TRAF3.
Fig. 6A shows the increase of cellular F-actin content by T3BP.
Fig. 6B shows the results of observation of cellular distribution of F-actin in cells which express T3BP.

### Best Modes for Carrying out the Invention

The embodiments of the present invention and processes for carrying out them are described in detail below.

### (1) The protein and gene of the present invention

The present invention relates to a protein having any of the following amino acid sequences, and a gene encoding the same.
(a) an amino acid sequence of SEQ ID NO: 1;
(b) an amino acid sequence obtained by deletion, substitution, and/or insertion of one or several of the amino acids in the amino acid sequence of SEQ ID NO: 1 and having activity of binding to TRAF3; or
(c) an amino acid sequence having a homology of 60% or more to the amino acid sequence of SEQ ID NO: 1 and having activity of binding to TRAF3.

The examples of the gene of the present invention include a gene having any of the following nucleotide sequences:
(a) a nucleotide sequence of SEQ ID NO: 2;
(b) a nucleotide sequence obtained by deletion, addition, or substitution of one or several of the nucleotides in the nucleotide sequence of SEQ ID NO: 2 and encoding a protein having activity of binding to TRAF3; or
(c) a nucleotide sequence which hybridizes with the nucleotide sequence of SEQ ID NO: 2 under stringent conditions and encoding a protein having activity of binding to TRAF3.

The range of "one or several" in the aforementioned " an amino acid sequence obtained by deletion, substitution, and/or insertion of one or several of the amino acids in the amino acid sequence of SEQ ID NO: 1" is not particularly limited. For example, it means approximately 1 to 20, preferably 1 to 10, more preferably 1 to 7, further preferably 1 to 5, and particularly preferably 1 to 3.

The aforementioned " an amino acid sequence having a homology of 60% or more to the amino acid sequence of SEQ ID NO: 1" means that the homology of said amino acid sequence to the amino acid sequence of SEQ ID NO: 1 is at least 60%, preferably at least 70%, more preferably at least 80%, further preferably at least 90%, particularly preferably at least 95%, and most preferably at least 98%.

The range of "one or several" in the aforementioned " a nucleotide sequence obtained by deletion, addition, or substitution of one or several of the nucleotides in the nucleotide sequence of SEQ ID NO: 2" is not particularly limited. For example, it is approximately 1 to 60, preferably 1 to 30, more preferably 1 to 20, further preferably 1 to 10, and particularly preferably 1 to 5.

The aforementioned "a nucleotide sequence which hybridizes under stringent conditions" refers to a nucleotide sequence of DNA which is obtained by colony hybridization, plaque hybridization, Southern blot hybridization or the like using DNA as a probe. An example of DNA is DNA that can be identified by hybridization using a filter comprising colony- or plaque-derived DNA or a fragment thereof immobilized thereon in the presence of 0.7 to 1.0M NaCl at 65°C, followed by washing of the filter using a 0.1 to 2 x SSC solution (1 x SSC solution is 150 mM sodium chloride and 15 mM sodium citrate) at 65°C. Hybridization can be carried out in accordance with a process described in, for example, Molecular Cloning: A laboratory Mannual, 2^{nd} Ed., Cold Spring Harbor Laboratory, Cold Spring Harbor, NY., 1989 (hereinafter abbreviated as "Molecular Cloning, 2^{nd} Ed.").

Examples of DNA that hybridize under stringent conditions include those having homology of certain level or higher to the nucleotide sequence of DNA that is used as a probe. For example, such DNA has homology of at least 70%, preferably at least 80%, more preferably at least 90%, further preferably at least 93%, particularly preferably at least 95%, and most preferably at least 98%.

The aforementioned "having activity of binding to TRAF3" refers to that the protein can bind to TRAF3 with an affinity equivalent to or higher than that of a protein having the amino acid sequence of SEQ ID NO: 1, and can thus be involved in intracellular signal transduction via TRAF3.

A process for obtaining the gene of the present invention is not particularly limited. Based on the information on the nucleotide sequence and the amino acid sequence of SEQ ID NOs: 1 and 2 in the Sequence Listing in the specification, suitable probes or primers are prepared, and they are used to screen the cDNA library of mouse and the like. Thus, the gene of the present invention can be isolated.

The mouse-derived cDNA library is preferably prepared from cells, organs or tissues in which the gene of the present invention is expressed. An example of cells in which the aforementioned gene is expressed is WEHI231 cell, but is not limited thereto.

DNA having the nucleotide sequence of SEQ ID NO: 2 can be obtained by PCR. PCR is carried out using mouse chromosomal DNA or cDNA library as a template and using a pair of primers designed to amplify the nucleotide sequence of SEQ ID NO: 2.

The reaction conditions for PCR can be suitably selected. For example, a cycle of reaction of denaturation at 94°C for 30 seconds, annealing at 55°C for 30 seconds to 1 minute and elongation at 72°C for 2 minutes is repeated, for example, 30 times, followed by a reaction at 72°C for 7 minutes. Subsequently, the amplified DNA fragment can be cloned into a suitable vector that can be amplified in a host such as *E*. *coli.*

Procedures such as the aforementioned preparation of probes or primers, construction of the cDNA library, screening of the cDNA library, and cloning of the gene of interest are known to persons skilled in the art. For example, these procedures can be carried out in accordance with a process described in Molecular Cloning, 2^{nd} Ed. or Current Protocols in Molecular Biology, Supplement 1 to 38, John Wiley & Sons (1987-1997) (hereinafter abbreviated as "Current Protocols in Molecular Biology").

The gene having a nucleotide sequence obtained by deletion, addition or substitution of one or several of the nucleotides in the nucleotide sequence of SEQ ID NO: 2 and encoding a protein having activity of binding to TRAF3 described in (b) above (variant gene) can be prepared by any process known to persons skilled in the art such as chemical synthesis, genetic engineering, and mutagenesis. Specifically, DNAs having a nucleotide sequence of SEQ ID NO: 2 are used, and mutations are introduced in these DNAs to obtain mutant DNAs.

For example, this can be realized by subjecting DNA having a nucleotide sequence of SEQ ID NO: 2 to a process in which the DNA is brought into contact with a mutagenic agent, a process in which the DNA is irradiated with ultraviolet rays, or genetic engineering. Site-directed mutagenesis, which is one method of genetic engineering, is useful since this process realizes the introduction of a specific mutation into a specific site. This process can be carried out in accordance with a method described in, for example, Molecular cloning, 2^{nd} Ed. or Current Protocols in Molecular Biology.

The nucleotide sequence that hybridizes with the nucleotide sequence of SEQ ID NO: 2 under stringent conditions and encodes a protein having activity of binding to TRAF3 described in (c) above can be obtained by colony hybridization, plaque hybridization, Southern blot hybridization or the like under certain hybridization conditions as mentioned above.

Processes for obtaining and producing the protein of the present invention are described below. Processes for obtaining and producing the protein of the present invention are not particularly limited, and any of naturally-occurring proteins, chemically synthesized proteins or recombinant proteins produced by gene recombination techniques can be used. Recombinant proteins are preferable in terms of mass-producibility with relatively simple operations.

When naturally-occurring proteins are obtained, proteins can be isolated from cells or tissues which express these proteins by suitable combinations of several techniques for isolating and purifying proteins. For obtaining chemically synthesized proteins, the protein of the present invention can be synthesized by chemical synthesis techniques such as the Fmoc (fluorenylmethyloxycarbonyl) method or the tBoc (t-butyloxycarbonyl) method. Various peptide synthesizers commercially available from, for example, Sowa Trading Co., Inc. (Advanced Chem Tech, USA), Perkin Elmer Japan (Perkin Elmer, USA), Pharmacia Biotech (Pharmacia Biotech, Sweden), Aloka Co. (Protein Technology Instrument, USA), Kurabo Industries Ltd. (Synthecell-Vega, USA), Nihon PerSeptive Ltd. (PerSeptive, USA), or Shimadzu, can be used to synthesize the protein of the present invention.

In order to produce the protein of the present invention as a recombinant protein, DNA having a nucleotide sequence encoding the protein (e.g., the nucleotide sequence of SEQ ID NO: 2), a variant thereof or a homolog thereof is obtained, and this is introduced into a suitable expression system, thereby producing the protein of the present invention. Production of expression vectors and transformants as well as production of recombinant proteins using the same are described later in this description.

A protein having an amino acid sequence obtained by deletion, substitution, or insertion of one or several of the amino acids in the amino acid sequence of SEQ ID NO: 1 or a protein having an amino acid sequence having a homology of 60% or more to the amino acid sequence of SEQ ID NO: 1 can be suitably produced or obtained by persons skilled in the art based on the information on the nucleotide sequence of SEQ ID NO: 2 which shows an example of the DNA sequence encoding the amino acid sequence of SEQ ID NO: 1.

For example, DNA can be isolated from a mouse or non-mouse organism by using a DNA probe having the nucleotide sequence of SEQ ID NO: 2 or a part thereof and screening for the homolog of the DNA under suitable conditions. Alternatively, a corresponding cDNA sequence is obtained by a homology search of a DNA sequence database, and a cDNA can be prepared by PCR. After the full-length DNA of this homologous DNA is cloned, and is incorporated into an expression vector and expressed in a suitable host. Thus, a protein that is encoded by the homologous DNA can be produced.

### (2) Vectors having the gene of the present invention

The gene of the present invention can be incorporated into a suitable vector to be used as a recombinant vector. A vector may be an expression vector or an non-expression vector, and can be selected in accordance with the desired purpose.

Preferably, a cloning vector is autonomously replicable in *E*. *coli* K12, and any vector such as a phage vector or plasmid vector can be used. Specific examples thereof include ZAP Express (Stratagene, Strategies, 5, 58 (1992)), pBluescript II SK(+) (Nucleic Acids Research, 17, 9494 (1989)), Lambda ZAP II (Stratagene), λgt10, λgt11 (DNA Cloning, A Practical Approach, 1, 49 (1985)), λTriplEx (Clontech), λExCell (Pharmacia), pT7T318U (Pharmacia), pcD2 (Mol. Cell. Biol., 3, 280 (1983)), pMW218 (Wako Pure Chemical Industries, Ltd.), pUC118 (Takara Shuzo Co., Ltd.), pEG400 (J. Bac., 172, 2392 (1990)), and pQE-30 (QIAGEN).

Preferably, an expression vector can be autonomously replicated in a host cell or incorporated into a chromosome of the host cell. An expression vector which comprises a promoter in such a site that the gene of the present invention can be expressed, can be used.

When bacteria are used as host cells, it is preferred that an expression vector for the gene of the present invention is autonomously replicable in the bacteria and, at the same time, is a recombinant vector composed of a promoter, a ribosome binding sequence, the aforementioned DNA and a transcription termination sequence. It may also contain gene which controls a promoter.

Examples of expression vectors for bacteria include pBTrp2, pBTac1 pBTac2 (commercially available from Boehringer Mannheim), pKK233-2 (Pharmacia), pSE280 (Invitrogen), pGEMEX-1 (Promega), pQE-8 (QIAGEN), pQE-30 (QIAGEN), pKYP10 (JP Patent Publication (Unexamined Application) No. 58-110600), pKYP200 (Agric. Biol. Chem., 48, 669 (1984)), pLSA1 (Agric. Biol. Chem., 53, 277 (1989)), pGEL1 (Proc. Natl. Acad. Sci. USA, 82, 4306 (1985)), pBluescriptII SK(+), pBluescriptII SK(-) (Stratagene), pTrS30 (FERM BP-5407), pTrS32 (FERM BP-5408), pGEX (Pharmacia), pET-3 (Novagen), pTerm2 (US 4686191, US 4939094, US 5160735), pSupex, pUB110, pTP5, pC194, pUC18 (Gene, 33, 103 (1985)), pUC19 (Gene, 33, 103 (1985)), pSTV28 (Takara Shuzo Co., Ltd.), pSTV29 (Takara Shuzo Co., Ltd.), pUC118 (Takara Shuzo Co., Ltd.), and pQE-30 (QIAGEN). Examples of promoters for bacteria include promoters derived from *E*. *coli* or phages such as trp promoter (Pₜᵣₚ), lac promoter (P_{lac}), P_{L} promoter, P_{R} promoter, and P_{SE} promoter, SPO1 promoter, SPO2 promoter, and penP promoter.

Examples of expression vectors for yeast include YEp13 (ATCC37115), YEp24 (ATCC37051), YCp50 (ATCC37419), pHS19, and pHS15. Examples of promoters for yeast include PHO5 promoter, PGK promoter, GAP promoter, ADH promoter, gal 1 promoter, gal 10 promoter, heat shock protein promoter, MFa1 promoter, and CUP1 promoter.

Examples of expression vectors for animal cells include pcDNAI, pcDM8 (commercially available from Funakoshi), pAGE107 (JP Patent Publication (Unexamined Application) No. 3-22979; Cytotechnology, 3, 133, (1990)), pAS3-3 (JP Patent Publication (Unexamined Application) No. 2-227075), pCDM8 (Nature, 329, 840, (1987)), pcDNAI/Amp (Invitrogen), pREP4 (Invitrogen), pAGE103 (J. Biochem., 101, 1307 (1987)), and pAGE210. Examples of promoters for animal cells include Cytomegalovirus (human CMV) immediate early (IE) gene promoter, SV40 early promoter, retrovirus promoter, metallothionein promoter, heat shock promoter, and SRa promoter.

Examples of expression vectors for plant cells include pIG121-Hm (Plant Cell Report, 15, 809-814 (1995)) and pBI121 (EMBO J. 6, 3901-3907 (1987)). Examples of promoters for plant cells include cauliflower mosaic virus 35S promoter (Mol. Gen. Genet (1990), 220, 389-392) and ribulose bisphosphate carboxylase small subunit promoter.

### (3) Transformant that has the gene of the present invention

A transformant that has the gene encoding a protein having activity of binding to TRAF3 according to the present invention can be produced by introducing the aforementioned recombinant vector (preferably an expression vector) into a host.

Examples of bacterial host cells include microorganisms belonging to the genus Escherichia, *Corynebacterium, Brevibacterium, Bacillus, Microbacterium, Serratia, Pseudomonas, Agrobacterium, Alicyclobacillus, Anabaena, Anacystis,* Arthrobacter, *Azobacter, Chromatium, Erwinia, Methylobacterium, Phormidium, Rhodobacter, Rhodopseudomonas, Rhodospirillum, Scenedesmun, Streptomyces, Synnecoccus,* or Zymomonas. Examples of processes for introducing a recombinant vector into a bacterial host include a method using calcium ions and a protoplast method.

Examples of yeast hosts include *Saccharomyces cerevisae, Schizosaccharomyces pombe, Kluyveromyces lactis, Trichosporon pullulans, and Schwanniomyces alluvius.* Examples of processes for introducing a recombinant vector into a yeast host include electroporation, spheroplast, and the lithium acetate method.

Examples of animal host cells include Namalwa cell, COS1 cell, COS7 cell, and CHO cell. Examples of processes for introducing a recombinant vector into an animal cell that can be used include electroporation, calcium phosphate method, and lipofection.

When an insect cell is used as a host cell, a recombinant gene-introducing vector and a baculovirus are introduced together into the insect cell, a recombinant virus is obtained in a culture supernatant of the insect cell, and then the insect cell is infected with the recombinant virus, thereby expressing a protein (described in, for example, Baculovirus Expression Vectors, A Laboratory Manual and Current Protocols in Molecular Biology, Bio/Technology, 6, 47 (1988)).

Examples of baculoviruses which can be used include the *Autographa californica* nuclear polyhedrosis virus, which infects an insect belonging to the Mamestra.

Examples of insect cells which can be used include ovarian cells of *Spodoptera frugiperda,* Sf9 and Sf21 (Baculovirus Expression Vectors, A Laboratory Manual, W. H. Freeman and Company, New York, 1992), and an ovarian cell of *Trichoplusia ni,* HiFive (Invitrogen).

Examples of processes for introducing the recombinant gene-introducing vector and the baculovirus together into the insect cell for preparing the recombinant virus include the calcium phosphate method and the lipofection method.

### (4) Production of recombinant protein using the transformant of the present invention

In the present invention, a transformant having the gene of the present invention prepared in the aforementioned manner is cultured, the protein of the present invention is produced and accumulated in the culture product, and the protein of the present invention is collected from the culture product. Thus, a recombinant protein can be isolated.

When the transformant of the present invention is a procaryotic microorganism such as E. *coli* or a eucaryotic microorganism such as yeast, a medium for culturing these microorganisms may be natural or synthetic so long as it contains carbon sources, nitrogen sources, inorganic salts, etc. assimilable by the microorganisms and can efficiently culture a transformant. Culturing is preferably carried out under aerobic conditions such as shaking culture or deep aeration agitation culture. Culturing temperature is generally 15°C to 40°C, and culturing time is generally 16 hours to 7 days. During the culturing, the pH is maintained at 3.0 to 9.0. The pH is adjusted with an inorganic or organic acid, an alkali solution, urea, calcium carbonate, ammonia, or the like. During the culturing, an antibiotic such as ampicillin or tetracycline may be added to the medium, if necessary.

Examples of a usable medium for culturing a transformant obtained from an animal host cell include the commonly used RPMI 1640 medium (The Journal of the American Medical Association, 199, 519 (1967)), Eagle's MEM medium (Science, 122, 501 (1952)), DMEM medium (Virology, 8, 396 (1959)), 199 medium (Proceeding of the Society for the Biological Medicine, 73, 1 (1950)), and a medium obtained by adding fetal bovine serum added to the above mediums. Usually, culturing is carried out at pH 6 to 8 at 30°C to 40°C in the presence of 5% CO₂ for 1 to 7 days. During culturing, an antibiotic such as kanamycin or penicillin may be added to the medium, if necessary.

An example of a usable medium for culturing a transformant obtained from a plant host cell is a commonly used medium selected depending on the relevant plant species, such as MS medium or R2P medium. Usually, culturing is carried out at pH 6 to 8 at 15°C to 35°C for 1 to 21 days. During culturing, an antibiotic such as kanamycin or hygromycin may be added to the medium, if necessary.

The protein of the present invention may be isolated and purified from a cultured transformant using a conventional technique for isolating and purifying proteins.

For example, when the protein of the present invention is expressed in a dissolved state in cells, cells are collected by centrifugation after the completion of culturing. The collected cells are suspended in an aqueous buffer, and crushed using an ultrasonic crusher, French press, Mantno-gaulin homogenizer, Dyno-mill, or the like to obtain a cell-free extract. This cell-free extract is centrifuged, and a supernatant is obtained. A purified sample can be obtained from the resulting supernatant by a conventional technique for isolating and purifying proteins, which includes solvent extraction, salting-out using ammonium sulfate, etc., desalting, precipitation using an organic solvent, anion exchange chromatography using resins such as diethylaminoethyl (DEAE) sepharose, DIAION HPA-75 (Mitsubishi Kasei), etc., cation exchange chromatography using resins such as S-Sepharose FF (Pharmacia), hydrophobic chromatography using resins such as butyl sepharose, phenyl sepharose, etc., gel filtration using a molecular sieve, affinity chromatography, chromatofocusing, and electrophoresis such as isoelectric focusing. These techniques may be used singly or in combinations of two or more.

When the protein is expressed with the formation of insoluble matter in cells, cells are similarly collected, crushed, and centrifuged. The protein is collected from the resulting precipitated fractions by a conventional technique, and the insoluble matter of the protein is solubilized with the aid of a protein denaturing agent. The solubilized liquid is diluted with or dialyzed with a solution containing no protein denaturing agent or a solution containing a protein denaturing agent diluted to the extent that it does not denature the protein. The protein is then made into a normal three-dimensional structure. Thereafter, a purified sample can be obtained by a technique for isolation and purification similar to that mentioned above.

### (5) Antibody which recognizes the protein of the present invention

The TRAF3-binding protein was identified by the present invention. Accordingly, an antibody which recognizes this protein can be prepared. Preparation of such an antibody is useful in research aiming at the elucidation of the dynamics of the aforementioned protein or TRAF3. The antibody of the present invention may be polyclonal or monoclonal, and it can be prepared by a conventional technique.

For example, a polyclonal antibody which recognizes the protein of the present invention can be obtained by immunizing a mammalian animal with the protein of the present invention as an antigen, collecting blood from the mammalian animal, and separating and purifying an antibody from the sampled blood. For example, a mammalian animal such as a mouse, hamster, guinea pig, chicken, rat, rabbit, dog, goat, sheep, or cow can be immunized. A process for immunization is known to persons skilled in the art, and immunization can be carried out by, for example, one or more administrations of an antigen. For example, an antigen may be administered two or three times at intervals of 7 to 30 days. The dose of an antigen can be, for example, about 0.05 to 2 mg per dose. The route of administration is not particularly limited, and hypodermic, intracutaneous, intraperitoneal, intravenous, or intramuscular administration can be suitably selected. Preferable administration is made by intravenous, intraperitoneal, or hypodermic injection. Also, an antigen can be dissolved in a suitable buffer, for example, a suitable buffer comprising complete Freund's adjuvant or a commonly used adjuvant such as aluminum hydroxide. It may be decided not to use an adjuvant depending on the route of administration or other relevant conditions.

The immunized mammalian animal is bred for a certain period of time, and the serum of the mammalian animal is then sampled to assay the antibody titer. When the antibody titer is elevated, additional immunization is carried out using, for example, 100 µg to 1000 µg of antigen. One to two months after the final administration, blood is sampled from the immunized mammalian animal, and the sampled blood is separated and purified by conventional techniques such as centrifugation, precipitation using ammonium sulfate or polyethylene glycol, or chromatography such as gel filtration chromatography, ion exchange chromatography, or affinity chromatography. Thus, a polyclonal antibody that recognizes the protein of the present invention can be obtained as a polyclonal antiserum. The complement system of the serum may be inactivated by treating the serum, for example, at 56°C for 30 minutes.

The globulin class of the monoclonal antibody that recognizes the protein of the present invention is not particularly limited, and examples thereof include IgG, IgM, IgA, IgE, and IgD. The type of the cell strain that produces the monoclonal antibody of the present invention is not particularly limited. For example, a cell strain can be obtained as a hybridoma by cell fusion of an antibody-producing cell and a myeloma cell line. A hybridoma that produces the monoclonal antibody of the present invention can be obtained by a cell fusion as described below.

As an antibody-producing cell, spleen cell, lymph node cell, B-lymphocyte or other cell obtained from the immunized animal is used. As an antigen, the protein of the present invention or a partial peptide thereof is used. Mice, rats, or the like can be used as animals to be immunized, and an antigen is administered to these animals by a conventional technique. For example, a suspension or emulsified liquid of an adjuvant such as complete or incomplete Freund's adjuvant and the protein of the present invention as an antigen is administered to animals intravenously, hypodermically, intracutaneously, or intraperitoneally several times for immunization. For example, a spleen cell is obtained from the immunized animal as an antibody-producing cell, and the obtained spleen cell is fused with a myeloma cell by a known technique (G Kohler et al., Nature, 256, 495 (1975)), thereby preparing a hybridoma.

Examples of myeloma cell lines used in the cell fusion include mouse cell lines P3X63Ag8, P3U1, and Sp2/0. When cell fusion is performed, a cell-fusion accelerator such as polyethylene glycol or Sendai virus is used. When a hybridoma is selected after the cell fusion, a hypoxanthine-aminopterin-thymidine (HAT) medium is used in accordance with a conventional technique. A hybridoma obtained by the cell fusion is cloned by limiting dilution, etc. Further, screening is optionally carried out by enzyme immunoassay using the protein of the present invention. Thus, a cell line that produces a monoclonal antibody specifically recognizing the protein of the present invention can be obtained.

In order to produce a monoclonal antibody of interest from the thus obtained hybridoma, this hybridoma is cultured by conventional cell culture or ascites production, and the monoclonal antibody may be purified from the culture supernatant or ascites. The monoclonal antibody can be purified from the culture supernatant or ascites by a conventional technique. For example, ammonium sulfate fractionation, gel filtration, ion exchange chromatography, and affinity chromatography can be used in suitable combinations.

Examples of processes for immunoassaying the TRAF3-binding protein of the present invention using the monoclonal antibody of the present invention include enzyme immunoassay, radioimmunoassay, fluorescence immunoassay, and luminescence immunoassay.

A fragment of the aforementioned antibody is also within the scope of the present invention. Examples of antibody fragments include an F(ab')2 fragment and an Fab' fragment.

Further, a labeled antibody of the aforementioned antibody is also within the scope of the present invention. Specifically, the thus prepared antibody of the present invention can be labeled and then used. Types and processes for labeling the antibody are known to persons skilled in the art. Examples include enzyme labeling using horseradish peroxidase, alkaline phosphatase, etc., fluorescent labeling using fluorescein isothiocyanate (FITC), tetramethylrhodamine B isothiocyanate (TRITC), etc., labeling using a coloring material such as a colloidal metal or colored latex, affinity labeling using biotin, etc., and isotopic labeling using ¹²⁵I, etc. Analyses such as the enzymatic antibody method, immunohistostaining, immunoblotting, direct immunofluorescence, or indirect immunofluorescence using the labeled antibody of the present invention, can be carried out by any process known to persons skilled in the art.

### (6) Screening process that uses the protein of the present invention

The present invention further relates to a process for screening for a substance that promotes or suppresses functions of the TRAF3-binding protein of the present invention or an antibody thereof using such a protein or antibody. In this screening process, a substance that promotes or suppresses the intracellular signal transduction via TRAF3 is preferably selected. Such a screening system can be constructed, for example, as described below.

An expression vector having the gene of the present invention is constructed, and this expression vector is introduced into a suitable TRAF3-expressing host. In the resulting transformant, both TRAF3 and the protein of the present invention are expressed, and the intracellular signal transduction proceeds through interactions therebetween in the presence of a suitable ligand.

A test substance can be added to the thus constructed screening system. If the progress of the intracellular signal transduction is inhibited when a test substance is added, this test material can be selected as a candidate for a substance that inhibits the interaction between the protein of the present invention and TRAF3 (i.e., a substance that suppresses the function of the TRAF3-binding protein of the present invention). On the contrary, if the intracellular signal transduction is activated when a test substance is added, this substance can be selected as a candidate for a substance that activates interactions between the protein of the present invention and TRAF3 (i.e., a substance that accelerates the function of the TRAF3-binding protein of the present invention).

Persons skilled in the art can suitably select an expression vector, host cell, or the like for the above screening system, and those mentioned above may be used.

Any substance can be used as a test substance without particular limitation. Examples of a test substance are low molecular weight synthetic compounds, extracts from naturally-occurring substances, compound library, phage display library, or combinatorial library. A process for constructing a compound library is known to persons skilled in the art, and a commercially available compound library can also be used.

### (7) Medicine of the present invention

The TNF ligand family is known as one of the most versatile cytokines among those that induce cytotoxicity, antiviral activity, immunoregulatory activity, and various cellular reactions including transcriptional control of several types of genes. Cellular responses to the TNF ligand are related not only to normal biological reactions but also to potentiation or inhibition of apoptosis. Abnormality in apoptosis control can cause various diseases. Examples of diseases related to cell growth or diseases related to apoptosis inhibition include cancer, autoimmune disease, viral disease, inflammation, graft-versus-host disease, acute graft rejection, and chronic graft rejection. Examples of diseases related to potentiation of apoptosis include AIDS, neurodegenerative disease, osteomyelodysplasia syndrome, ischemic damage, toxin-induced hepatic failure, septic shock, cachexia, and asitia.

Although the TRAF3-binding protein of the present invention does not belong to the TNF receptor superfamily, it binds to TRAF3. Accordingly, it is considered to be related to chronic and acute inflammations, arthritis, sepsis, autoimmune diseases (e.g., systemic lupus erythematosus, chronic articular rheumatism, inflammatory intestinal disease, or psoriasis), graft rejection, graft-versus-host disease, infectious diseases, apoplectic seizure, ischemia, acute respiratory disease syndrome, restenosis, brain damage, AIDS, bone disease, cancer such as lymphocyte proliferative disease, atherosclerosis, Alzheimer's disease, and other diseases that are associated with the TNF receptor/ligand superfamily.

Accordingly, a substance that promotes or suppresses functions of the TRAF3-binding protein of the present invention obtained by the aforementioned screening process is useful as a therapeutic and/or preventive agent for these diseases.

Thus, the present invention also relates to a medicine that comprises, as an active ingredient, a substance that promotes or suppresses functions of the TRAF3-binding protein of the present invention obtained by the aforementioned screening process. For example, the medicine of the present invention can be used for preventing or treating diseases associated with abnormalities in intracellular signal transduction via TRAF3.

In general, the medicine of the present invention comprises, as an active ingredient, a substance that promotes or suppresses functions of the TRAF3-binding protein of the present invention, and it is provided in the form of a pharmaceutical composition comprising a pharmaceutical additive (for example, a carrier or excipient).

The route for administering the medicine of the present invention is not particularly limited. The medicine may be administered orally or parenterally (e.g., via intramuscular administration, intravenous administration, hypodermic administration, intraperitoneal administration, application to mucosa such as that in the nasal cavity, or inhalation).

The form of the medicine of the present invention is not particularly limited. Examples of forms of dosage for oral administration include tablets, capsules, subtle granules, powders, granules, liquids, and syrups. Examples of preparations for parenteral administration include injections, drops, suppositories, inhalants, transmucosal absorbents, transdermal absorbents, nasal drops, and ear drops.

Persons skilled in the art can suitably select the form of the medicine of the present invention, pharmaceutical additives to be used, processes for producing the medicine, and the like.

The dose of the medicine of the present invention can be suitably selected from comprehensive viewpoints of the sex, age, body weight of the patient, severity of symptoms, purpose of administration (whether it is preventive or therapeutic), and the presence or absence of other complications. The dose is generally 0.001 µg/kg of body weight/day to 1,000 µg/kg of body weight/day, and preferably 0.001 µg/kg of body weight/day to 100 µg/kg of body weight/day.

### (8) Diagnostic process for disease by measuring the level of the protein of the present invention

The present invention further relates to a diagnostic process for diseases associated with abnormalities in intracellular signal transduction via TRAF3, which comprises measuring the level of the protein of the present invention in an organism-derivedingredient.

As mentioned above, the protein of the present invention may be involved in various diseases associated with the potentiation or inhibition of apoptosis. Accordingly, the assay of an activity or level of the protein of the present invention in the organism enables the diagnosis of diseases associated with the abnormalities in intracellular signal transduction via TRAF3.

The level of the protein of the present invention can be measured in accordance with any conventional technique known to persons skilled in the art. For example, it may be carried out by an immunological process using an antibody that recognizes the protein of the present invention. Alternatively, it may be carried out by measuring the amount of mRNA encoding the protein of the present invention.

### Examples

The present invention is further illustrated by the following examples, but the present invention is not limited by the following examples.

### (A) Materials and Methods

### A-1. Yeast-based screening of protein-protein interaction.

DNA encoding full-length TRAF3 was used as bait in the CytoTrap^{TM} Two-hybrid System (Stratagene, La Jolla, CA). The yeast strain cdc25H (temperature sensitive mutant strain of cdc25 gene) used in this system has the following genotype; *MATa ura3, lys2, leu2, trp1, his200, ade101, cdc25-2, GAL*^{*+*}*.* A library of hybrid protein between myristylation signal sequence and cDNA fragments derived from WEHI231 cells, was constructed in plasmid pMry. The screening was performed according to the manufacturer's recommendations with little modification.

Briefly, the yeast strain was normally grown in YPAD medium or Burkholder's Minimum Media (BMM) fortified with appropriate supplements at 25°C [Sato, T. et al., (1995) FEBS Lett. 358, 113-118] and was transformed by lithium acetate method. The yeast transformants were grown on BMM/galactose plates at 25°C for 2 days and then were grown at 37°C. The colonies that grew at 37°C and were considered to express a myristylated protein which interacts with TRAF3, were pick up and tested for growth on BMM/glucose and BMM/galactose plates at 37°C. The library plasmids (pMry to which cDNA was integrated) were isolated from the clones that exhibited galactose-dependent growth (the expression of cDNA integrated in pMyr is dependent on galactose) at 37°C, and re-transformed into cdc25H cells with either the plasmid pSos/TRAF3 or pSos/collagenase as a negative control. The plasmids which suppressed the cdc25H phenotype only in the presence of pSos/TRAF3 were sequenced. DNA sequencing was performed on an Applied Biosystems automated DNA sequencer, ABI PRISM 310 Genetic Analyzer (Foster, CA).

### A-2. Northern blot hybridization

10 µg of total RNA prepared by acid guanidine phenol chloroform method from mouse tissues (C3H/Hen Crj) was separated on 1 % agarose gel containing formaldehyde, transferred to Hybond™ -N+ (Amersham Pharmacia, Uppsala, Sweden) in 20×SSC, and immobilized by UV-cross linking (Stratagene). A hybridization probe was prepared from gel-purified DNA fragment which was radio-labeled by random priming with [α ⁻³²P]dCTP (Amersham Pharmacia). The blot was hybridized in a solution containing 5×SSC, 5×Denhardt's solution, 0.1% SDS, 50% deionized formamide and 100 µg/ml salmon sperm DNA at 42°C. After hybridization, the blot was washed with 2×SSC and 0.1% SDS at room temperature and with 0.1×SSC and 0.1% SDS at 65°C. The blot was dried and analyzed with BAS2500 (Fuji Ltd., Tokyo, Japan).

### A-3. Construction of expression plasmid and transfection

To make T3BP expression constructs, the appropriate regions of mouse T3BP was amplified by PCR and subcloned into pcDNA 3.1 (-) myc his version B (Invitrogen, Carlsbad, CA) using *EcoRI* and XhoI restriction site, resulting in pcDNA/T3BP. T3BP was expressed as C-terminally tagged MYC fusion proteins.

For construction of EGFP-tagged T3BP expression plasmid, the full-length coding region was amplified by PCR and ligated into pEGFP-N2 (Clontech, California, CA) using *EcoRI* and *XhoI* restriction site, resulting in pEGFP/T3BP.

Mammalian expression vectors encoding FLAG-tagged mouse TRAF2 and TRAF3 were generated by PCR-based method.

All expression constructs were sequenced using ABI PRISM 310 Genetic Analyzer.

### A-4. Cell culture and transfection

Human embryo kidney 293 cells were maintained in Eagle's minimum essential medium containing 10% fetal calf serum (HyClone, Logan, UT), 100 U/ml penicillin, and 100 µg/ml streptomycin (GIBCO-BRL, Grand Island, NY). WEHI231 cells were maintained in RPMI-1640 medium containing 10% fetal calf serum (HyClone), 5.5×10⁻⁵ M 2-mercaptoethanol, 100 U/ml penicillin, and 100 µg/ml streptomycin (GIBCO-BRL). 293 cells were seeded at a density of 10⁶ cells/dish in 10-cm culture dishes and were cultured for 2-3 days. Then, the cells were transfected by standard calcium phosphate co-precipitation method using commercial solution (Eppendorf-5 Prime, Inc., Boulder, CO).

### A-5. GST pull-down assay

For GST pull-down assay, GST fusion proteins were expressed in *Escherichia coli* (BLR) after IPTG induction using pGEX vector (Amersham Pharmacia) and purified on glutathione beads (Amersham Pharmacia). FLAG-tagged TRAF2, TRAF3, TRAF5 and TRAF6 were expressed in 293 cells. The cell lysates were prepared from the transfected cell by using E1A Buffer (50 mM HEPES [pH7.6], 250 mM NaCl, 0.1% NP-40 and 5 mM EDTA) as lysis. The cell lysates were cleared and incubated with 2 µg GST or GST fusion protein immobilized on glutathione beads. The glutathione beads were washed extensively with E1A buffer. Samples were subjected to 10% SDS-polyacrylamide gel electrophoresis and transferred to PVDF membrane. After blocking, the membrane was treated with anti-FLAG M2 antibody and immunoreactive proteins were detected according to the enhanced chemiluminescence protocol (ECL, Amersham Pharmacia) using 1:5,000 horseradish peroxidase-linked secondary antibody (Bio-Rad). In vitro transcription and translation and ³⁵S methionine labeling of TRAF3 were carried out in accordance with T7 link transcription/translation system (Amersham Pharmacia), and the detection was carried out by fluorography.

### A-6. Immunoprecipitation assay

The cells were detached from the dishes in PBS containing 5 mM EDTA and washed three times with the same buffer. The cells were lysed in 1ml of E1A Buffer for 10 min at 4°C. The cell lysates were centrifuged and the supernatants were used as cell extracts. The cell extracts were incubated with anti-FLAG M2 antibody, anti-MYC 9E10 antibody or control antibody for 2 hours at 4°C, followed by treatment with protein G sepharose FF (Amersham Pharmacia). The sepharose beads were washed extensively with E1A buffer. Samples were subjected to 10% SDS-polyacrylamide gel electrophoresis and immunoreactive proteins were detected described above with anti-FLAG M2 antibody or anti-MYC 9E10 antibody.

### A-7. Fluorescence microscopy

293 cells cultured on cover glasses were transfected with various construct. After 24 hours, the cells were fixed in PBS containing 3.7% formalin for 10 minutes at room temperature. The cells were washed three times with PBS and treated with 0.2% Triton X-100 in PBS for 5 minutes at room temperature. After blocking with skim milk, the cells were incubated with various first antibodies such as anti-FLAG M2 monoclonal antibody or anti-MYC 9E10 antibody (Sigma), followed by Cy5-conjugated secondary antibody (Jackson ImmunoResearch Laboratories, Inc.). The cells were washed three times with PBS and mounted on slide glasses.

Fluorescence was visualized by using a Carl Zeiss LSM510 confocal laser scanning microscope (Oberkochen, Germany).

### A-8. Quantification of F-actin

F-actin content measurement was performed as described previously [Mizuno., et al., (1998) J.Pharm.Pharmacol. 50, 645-648]. Briefly, 293 cells cultured on 12-well plates were transiently transfected with the indicated amount of T3BP expression plasmid by calcium phosphate method. The total DNA (2 µg) transfected was kept constant by supplementation with empty vector. After 24 hours, the transfected cells were fixed with 3.7% formalin in PBS for 10 minutes at 37°C. After permeabilization with 0.2% Triton X-100 in PBS for 10 minutes at room temperature, the cells were stained with 10 unit/ml rhodamin-phalloidin (MolecurProbe) in PBS for 1 hour at room temperature. After extensive wash with PBS, the bound FITC-phalloidin was extracted with methanol on ice for 1 hour. The fluorescence intensity was measured by using a spectrometer, F-3010 (Hitachi Ltd., Tokyo, Japan) with excitation wave length of 488 nm and emission wave length of 510 nm. The results were expressed as the relative fluorescence intensity calculated from the ratio of that of control transfected cells.

### (B) Results

### B-1. Isolation of cDNA Clone Encoding T3BP

To identify the proteins that associate directly with TRAF3, a yeast-based protein-protein interaction screening was performed. *S. cerevisiae* cdc25H strain contains a temperature sensitive point mutation in the cdc25 gene, which prevents host growth at 37°C, but at permissive temperature (25°C), host growth is normal. The cdc25 gene encodes a guanyl nucleotide exchange factor, which binds and activates RAS, leading to cell growth. The system used in this example is based on the ability of hSos to complement the cdc25 defect and activate the yeast RAS signaling pathway; once hSos fusion protein (hSos-TRAF3) is expressed and localized to the plasma membrane through a protein-protein interaction, the cdc25H strain grows at 37°C.

The yeast containing plasmid pSos/TRAF3 was transformed with a WEHI231 cell cDNA expression library that fused to myristylation signal to locate the plasma membrane. Approximately 2×10⁵ transformants were grown on BMM/galactose plates at 25°C for 2 days and transferred into incubator at 37°C. The colonies that grew at 37°C were pick up and tested for growth on BMM/glucose and BMM/galactose plates at 37°C. Library plasmids were isolated from the clones that exhibited galactose-dependent growth at 37°C and re-transformed into cdc25H cells with either the pSos/TRAF3 or pSos/collagenase as a negative control. Finally, 9 cDNAs which suppressed the cdc25H phenotype only in the presence of pSos/TRAF3, were obtained. One of these clones, clone 3, encodes a portion of novel protein.

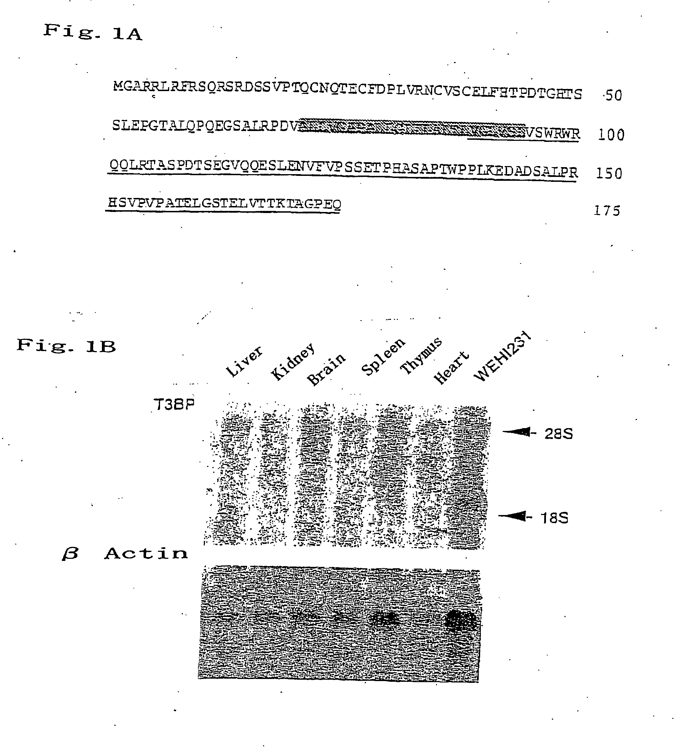

To isolate the full-length cDNA inserted into clone 3, a pMry cDNA library derived from WEHI231 cells was screened by using partial cDNA as a probe, and several positive clones were obtained. DNA sequence analysis of positive clones revealed an open reading frame predicted to encode a protein of 175 amino acids with an estimated molecular weight of 18,846 dalton (Fig. 1A; SEQ ID NOS. 1 and 2 of the Sequence Listing). This novel protein is designated as T3BP.

Fig. 1A shows the amino acid sequence deduced from the nucleotide sequence of the full-length T3BP cDNA. The sequence encoded by clone 3 is underlined. The shade box shows the putative transmembrane domain.

Database searches utilizing BLAST and FASTA programs revealed that T3BP cDNA contained the sequence which was reported as a somatically acquired provirus flanking sequence in mouse leukemia and lymphomas (AF193161). It was also revealed that human B-cell maturation factor (BCMA) had the significant sequence similarity to T3BP and shared an 28% identity and 42% similarity to T3BP. The hydropathy plot generated with the Tmpred program suggested that T3BP has a putative transmembrane region (amino acids 77-97). PROSITE analysis identified N-glycosylation site (amino acids 23-26) and N-myristoylation site (amino acids 81-86). PSORT analysis predicted that this protein had the type I membrane protein configuration and located at plasma membrane.

The expression pattern of T3BP was studied by Northern blot hybridization using 6 different tissues and WEHI231 cells. The results are shown in Fig. 1B. In Fig. 1B, the blot was hybridized with a 200b T3BP cDNA probe. Position of ribosomal RNAs are indicated on the left.

As shown in Fig. 1B, the T3BP gene showed an mRNA of approximately 2 kb in WEHI231 cells. However, T3BP mRNA could not be detected in other mouse tissues examined.

### B-2. T3BP Specifically Interacts with TRAF3.

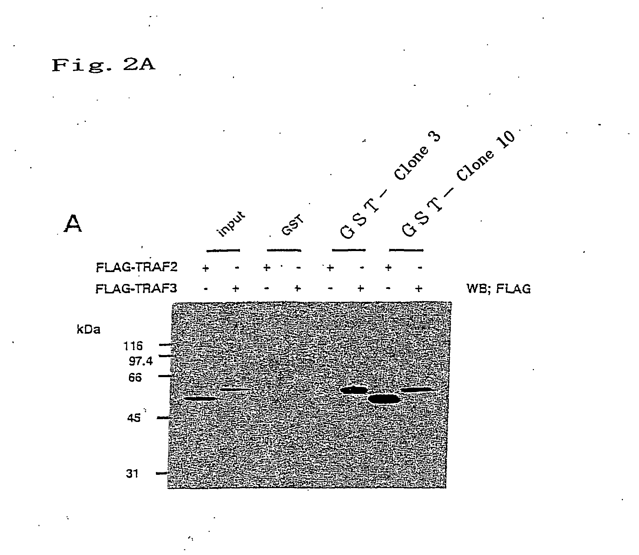

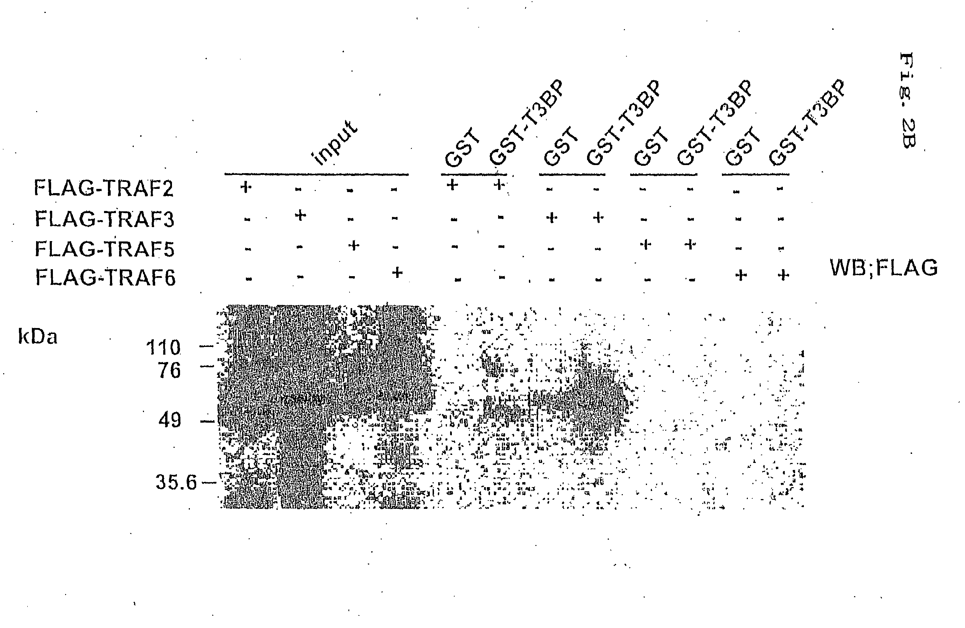

To confirm the interaction between TRAF3 and T3BP observed in yeast based assay, clone 3 and clone 10 were expressed as GST fusion proteins and its binding to TRAF2 or TRAF3 was examined in a GST pull-down assay. The cell lysates from 293 cells overexpressing FLAG-tagged TRAF2 or TRAF3 were incubated with GST-clone 3, GST-clone 10 or GST alone and TRAFs bound to GST fusion proteins were precipitated with glutathion-sepharose. The TRAFs were resolved by SDS-PAGE and detected by immunoblotting by using anti-FLAG M2 antibody. The expression levels of FLAG-tagged TRAFs in the cell lysates were also monitored by immunoblotting with anti-FLAG M2 antibody. The results are shown in Fig. 2A.

As shown in Fig. 2A, GST-clone 3 preferably associated TRAF3 rather than TRAF2. On the other hand, GST-clone 10 interacted with TRAF2 and TRAF3, equally. Sequence analysis of clone 10 indicated that this clone coded a part of mnb protein kinase homologue mp86 (Dyrk; MMU58497, amino acids 284-763), which contained the consensus sequence of TRAF binding domain (PXQXT/S, amino acids 583-587). However, clone 3 does not have this sequence.

Further, in order to compare the binding specificities of clone 2 and other TRAF families, the similar pull down assay was carried out. As shown in Fig. 2B, GST-T3BP (Clone 3) most strongly bound to TRAF3 as compared with other TRAF.

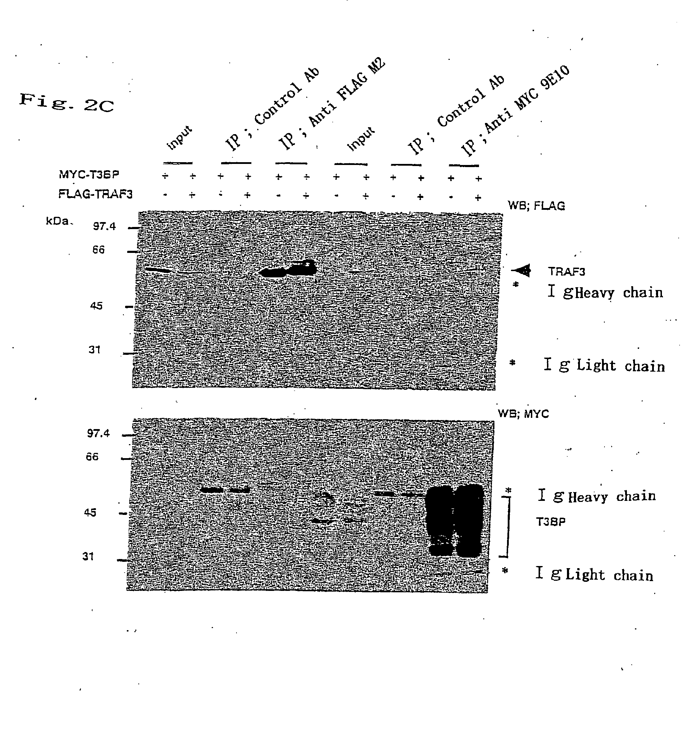

The interaction of T3BP with TRAF3 was also analyzed by co-immunoprecipitation assay in mammalian cells. The results are shown in Fig.2C. First, 293 cells were transiently transfected with expression vectors encoding MYC-tagged T3BP (full length T3BP containing C-terminal MYC epitope label) and the FLAG-tagged TRAF3. Cell extracts were prepared and immunoprecipitated (IP) with anti-MYC 9E10 antibody, anti-FLAG M2 antibody (anti-FLAG monoclonal antibody) or control mouse IgG. Co-precipitating FLAG-TRAF3 or MYC-T3BP was detected by immunoblotting analysis with the anti-FLAG M2 antibody (Upper of Fig. 2C) or anti-MYC 9E10 antibody (Lower of Fig. 2C), respectively. The expression level of T3BP and TRAF3 in the cell lysates were also determined by immunoblotting with anti-MYC 9E10 antibody or anti-FLAG M2 antibody, respectively. In Fig. 2C, The positions of T3BP and TRAF3 are indicated. The positions of Ig heavy and light chain were also indicated.

As shown in Fig.2C lower, anti-MYC antibody specifically recognized multiple bands in the lysates from the transfected cells with MYC-tagged T3BP expression plasmid, but not in the case of control plasmid. The size of bands (35-50 kDa) was bigger than that estimated from the amino acid sequence. The control antibody did not recognize the band. T3BP which precipitates with FLAG-tagged TRAF3 was detected at the position of about 40 kDa (lower). Reciprocally, TRAF3 which precipitates with MYC-tagged T3BP was detected at the position of about 64 kDa (upper).

### B-3. Mapping of T3BP binding domain in TRAF3.

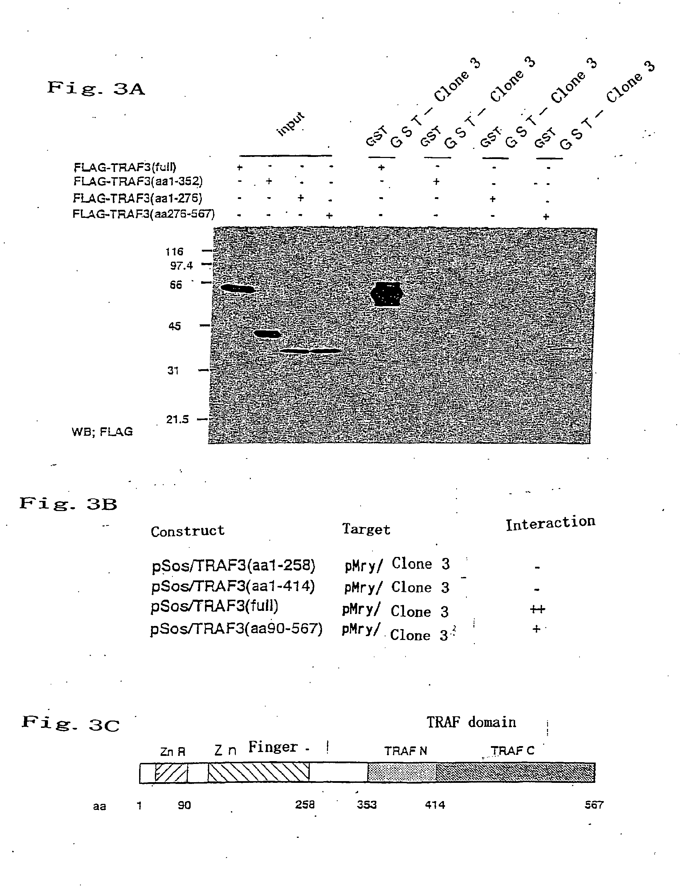

To determine which regions of TRAF3 contribute to the binding with T3BP, various truncation mutants of FLAG-tagged TRAF3 were expressed in 293 cells, and GST pull-down assay was performed. First, 293 cells were transiently transfected with the FLAG-tagged TRAF3 truncation mutants. Cell extracts were prepared and incubated with GST-clone 3 or GST alone. TRAFs bound to GST fusion proteins were precipitated with glutathion-sepharose, resolved by SDS-PAGE and detected by immunoblotting by using anti-FLAG M2 antibody. The expression levels of FLAG-tagged TRAF3 truncation mutants in the cell lysates were monitored by immunoblotting with anti-FLAG M2 antibody. The results are shown in Fig. 3A. As is understood from Fig. 3A, it was found that the TRAF domain of TRAF3 was needed to interaction with T3BP.

To confirm this, TRAF3 truncation mutant fused to hSos were generated and tested for interaction with T3BP in the yeast based protein-protein interaction assay. Specifically, yeast cells, cdc25H, were transfected with the indicated constructs and assayed for growth as described in method. The colonies that grew at over permissive temperature are positive and colonies that fail to grow at over permissive temperature are negative (Fig. 3B). From this assay, it is also suggested that TRAF domain is necessary for T3BP binding. Fig. 3C shows schematic presentation of primary structure of murine TRAF3.

### B-4. Mapping of TRAF3-binding domain in T3BP

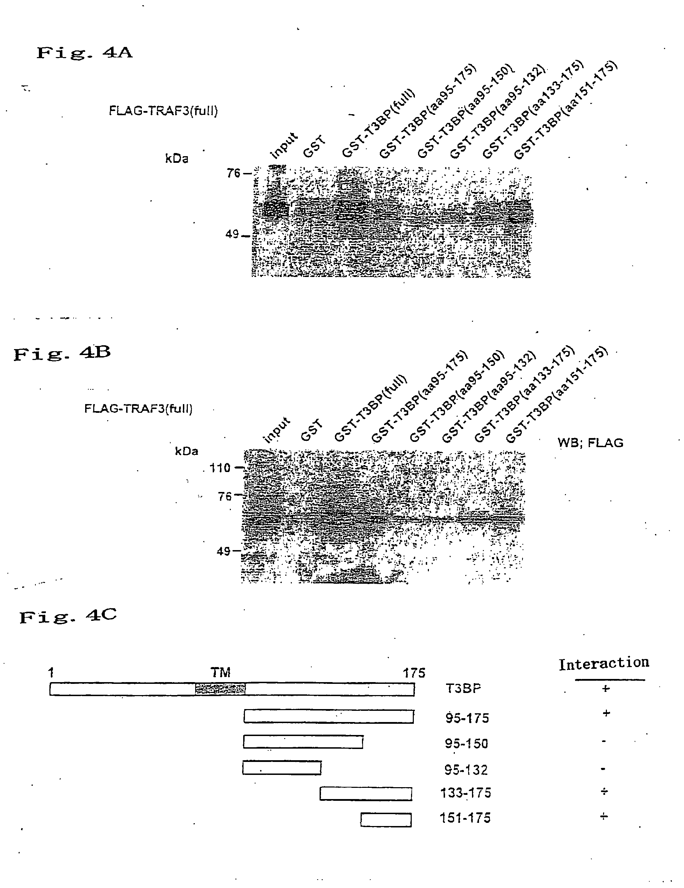

In order to determine the domain of T3BP that is involved in the binding with TRAF3, various truncation variants of T3BP were expressed in *E*. *coli* as GST fusion proteins, transcribed and translated *in vitro,* and incubated with ³⁵S-methionine-labeled TRAF3. T3BP that bound to the GST fusion proteins was precipitated with glutathione sepharose, separated by SDS-PAGE, and then detected by fluorography. As shown in Fig. 4A, the C-terminal portion containing amino acids 151 or higher in T3BP was found to be essential in the interaction with TRAF3. Further, FLAG-tagged TRAF3 was expressed in 293 cells, and the 293 cells lysate was subjected to GST pull-down assay using GST and a GST-fused T3BP truncation variant. As a result, the binding pattern detected by the anti-FRAG M2 antibody as shown in fig. 4B was in complete conformity with the binding test using an *in vitro* transcription and translation product as shown in Fig. 4A. Fig. 4C shows the primary structure of T3BP and its TRAF3-binding domain ("TM" indicates a transmembrane domain).

### B-5. Cellular distribution of T3BP

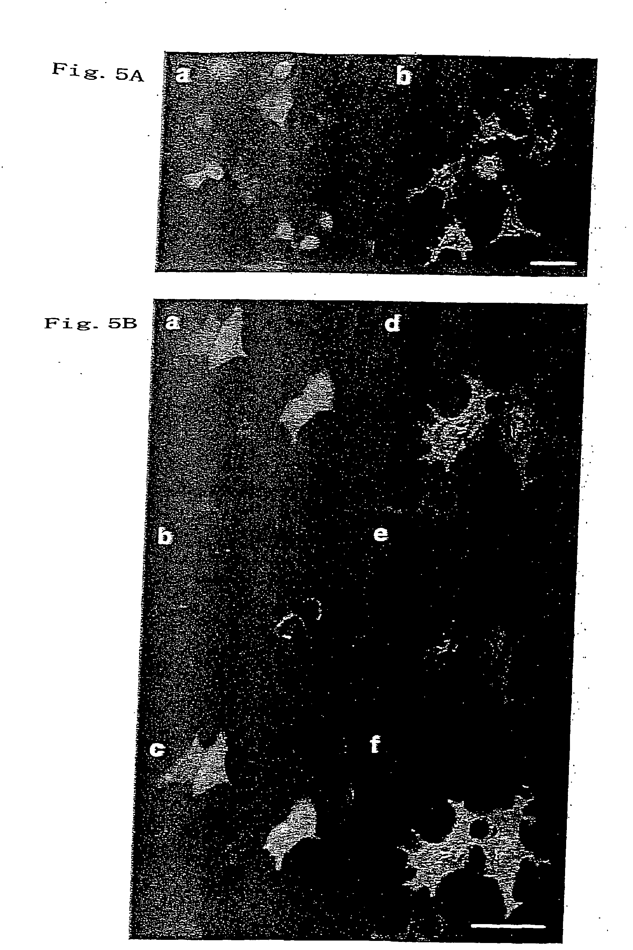

To examine the function of T3BP, its subcellular localization was examined by fluorescence microscopy. 293 cells cultured on cover glasses were transfected with pEGFP-N2 (control plasmid; left) or pEGFP/T3BP (EGFP labeled T3BP expression plasmid; right). After 24 hours, the cells were fixed in 3.7% formalin. Then, the fluorescence from EGFP was imagined with a confocal laser scanning microscope. The results are shown in Fig. 5A.

As shown in Fig. 5A, expression of EGFP-tagged T3BP induced morphological changes. In EGFP-tagged T3BP expressed cells, the cell shape became adhesive and the many spikes were grown from the cell surface. The almost fluorescence of EGFP-fused T3BP was observed at the cell surface and spikes. This observation consists with the prediction by PSORT.

Next, effect of T3BP expression on TRAF3 cellular distribution was examined. 293 cells cultured on cover glasses were transfected with pEGFP-N2 and pCR/FLAG-TRAF3 (a,b,c of Fig.5B) or pEGFP/T3BP and pCR/FLAG-TRAF3 (d,e,f of Fig.5B). After 2 days, the cells were fixed in 3.7% formalin and treated with 0.2% Triton X-100. After blocking, an indirect immunofluorescence analysis was performed. Anti-FLAG M2 antibody was used as a first antibody followed by the Cy5-conjugated secondary antibody teatment. Fluorescence from EGFP and Cy5 was imagined with a confocal laser scanning microscope. Upper; Fluorescence images of EGFP, Middle; Fluorescence images of Cy5-conjugated secondary antibody, Bottom; Merged images. Bar, 50 µm

### B-6. Effect of T3BP on Cellular F-actin content

To examine the effect of T3BP on cellular F-actin, the cellular content of F-actin in pcDNA/T3BP transfected cells was measured. 293 cells cultured on 12 well plates were transfected with the indicated amount of pcDNA/T3BP. After 24 hours, F-actin level was measured with FITC-phalloidin as described in method.

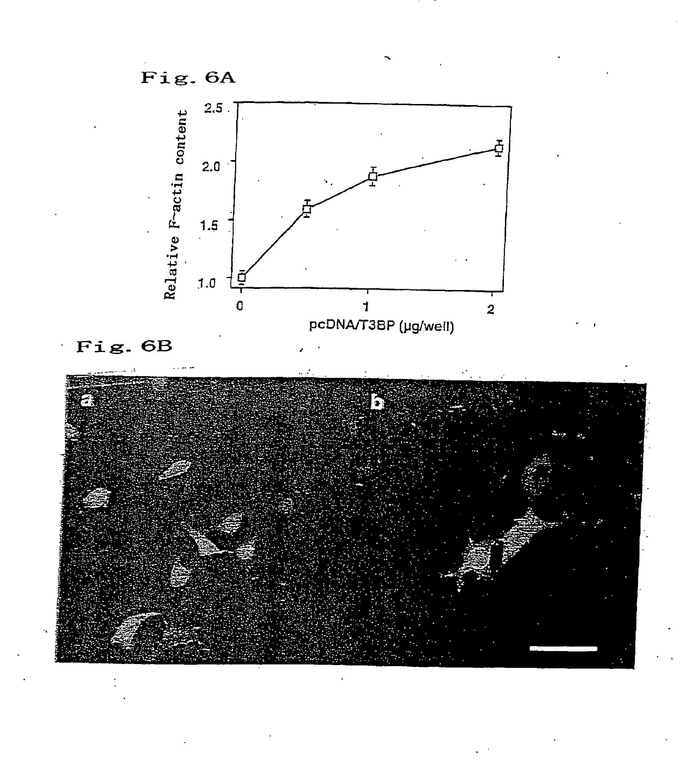

The results are shown in Fig. 6A. As is understood from Fig. 6A, T3BP increased the cellular F-actin content in a concentration dependent fashion.

To confirm that the cellular F-actin content is increased by T3BP, cellular distribution of F-actin in the cells expressing MYC-tagged T3BP was observed (Fig. 6B). Specifically, 293 cells cultured on cover glasses were transfected with pEGFP-N2 and pcDNA (a of Fig. 6B) or pEGFP-N2 and pcDNA/MYC-T3BP (b of Fig. 6C). After 24 hours, the cells were fixed in 3.7% formalin and treated with 0.2% Triton X-100. After blocking, the cells were stained with rhodamine-phalloidin. Fluorescence from EGFP (green) and rhodamine (red) was imagined with a confocal laser scanning microscope. Bar: 50µm

As is understood from b of Fig. 6B, MYC-tagged T3BP expression also caused change in cell shape and the spikes induced by T3BP were stained with rhodamine-phalloidin.

### (C) Conclusion

In this example, a novel TRAF3-binding protein, T3BP, was identified by using a yeast-based protein-protein interaction screening. The association between T3BP and TRAF3 was confirmed by both GST pull-down assay and co-immunoprecipitation assays (Fig. 2C). Moreover, T3BP and TRAF3 were co-located in the both proteins co-expressing cells (Fig. 5B). T3BP preferably interacted with TRAF3 among the TRAF family. Recent reports revealed the TRAF-binding proteins that specifically interact with a single member of TRAF family [Gamper, C., et al., (2000) Mol.Immunol. 37, 73-84; Ling, L., et al., (2000) J.Biol.Chem. 275, 23852-23860; and Ling, L., et al., (2000) Proc.Natl.Acad.Sci.USA 97, 9567-9572]. The MIP-T3 is identified as a TRAF3 specific TRAF-binding protein and links TRAF3 to microtubles as MIP-T3 binds to microtubles and recruits TRAF3 to microtubles (Ling, L., et al., (2000) J.Biol.Chem. 275, 23852-23860). Nucleoporin is also identified as a TRAF3 specific TRAF-binding protein (Gamper, C., et al., (2000) Mol.Immunol. 37, 73-84). Because it seems that TRAFs act redundantly or specifically in particular signaling cascade, identification and characterization of TRAF-binding proteins specific for single members of the TRAF family will be important for the different roles played by individual TRAF proteins.

T3BP has the significant sequence similarity to human BCMA, which belongs to TNFR superfamily as 17th member [Madry C., et al., (2000) Int.Immunol. 10, 1693-1702]. Interestingly, the expression of T3BP might be limited to a certain stage of B cell lineage as same as that of BCMA [Laabi, Y., et al., (1992) EMBO J. 11, 3897-3904; and Laabi, Y., et al., (1994) Nucleic Acid Res. 22, 1147-1154] and distributed at cell surface (Fig. 1B and Fig. 5B). Beside this, it is reported that T3BP gene was disrupted by murine leukemia retrovirous insertion in mouse leukemia and lymphomas [Hansen, GM., et al., (2000) Genome Res. 10, 237-43]. From these, there is a possibility that T3BP may play an important role in B cell differentiation as a cell surface receptor. However, T3BP does not belong to tumor necrosis factor receptor superfamily because T3BP does not has cystein-rich region in putative extracellular region.

A novel TRAF3-binding protein that was elucidated by the present invention was in conformity with a receptor [Thompson, J. S. et al., (2001), Science 293, 2108-2111] specific for BAFF that has been recently reported (B-cell activation factor from the TNF family (BAFF)). BAFF is associated with B-cell growth [Schneider, P. et al., (1999), J. Exp. Med. 189, 1747-1756], and a highly BAFF-expressing mouse exhibits mature B-cell hyperplasia or symptoms of systemic lupus erythematosus. Thus, the involvement thereof with autoimmune diseases caused by the growth of autoreactive B-cells is suggested [Mackay, F. et al., (1999), J. Exp. Med. 190, 1697-1710, Batten, M. et al., J. Exp. Med. (2000), 192, 1453-1466]. Accordingly, TRAF3 is deduced to have important functions in the intracellular signal transduction system for B-cell growth through BAFF. Therefore, it is expected that a therapeutic agent for autoimmune diseases is developed by means of the use of not only the regulation of the interaction between BAFF and a TRAF3-binding B-cell-specific receptor but also the regulation of the interaction between the TRAF3-binding B-cell-specific receptor and TRAF3.

### Industrial Applicability

According to the present invention, a novel signal transduction molecule that binds to TRAF3 was identified. Also, according to the present invention, a gene encoding a signal transduction molecule that binds to TRAF3 was cloned. The use of the novel protein identified by the present invention enables the development of a novel medicine or the provision of a diagnostic process for diseases.

## Claims

1. A protein having any of the following amino acid sequences:
(a) an amino acid sequence of SEQ ID NO: 1;
(b) an amino acid sequence obtained by deletion, substitution, and/or insertion of one or several of the amino acids in the amino acid sequence of SEQ ID NO: 1 and having activity of binding to TRAF3; or
(c) an amino acid sequence having a homology of 60% or more to the amino acid sequence of SEQ ID NO: 1 and having activity of binding to TRAF3.

2. A gene encoding the protein of the present invention.

3. A gene having any of the following nucleotide sequences:
(a) a nucleotide sequence of SEQ ID NO: 2;
(b) a nucleotide sequence obtained by deletion, addition, or substitution of one or several of the nucleotides in the nucleotide sequence of SEQ ID NO: 2 and encoding a protein having activity of binding to TRAF3; or
(c) a nucleotide sequence which hybridizes with the nucleotide sequence of SEQ ID NO: 2 under stringent conditions and encoding a protein having activity of binding to TRAF3.

4. A vector comprising the gene of claim 2 or 3.

5. A transformant having the gene of claim 2 or 3 or the vector of claim 4.

6. A process for producing a protein having activity of binding to TRAF3, wherein the transformant of claim 5 is used.

7. An antibody which recognizes the protein of claim 1.

8. A process for screening for a substance that promotes or suppresses functions of the protein or antibody, wherein the protein of claim 1, or TRAF3, or the antibody of claim 7 is used.

9. The process according to claim 8 wherein the protein comprises at least the sequence of 151th to 175th amino acids in the amino acid sequence of SEQ ID NO.1.

10. The process according to claim 8 wherein the TRAF3 comprises at least the sequence of 414th to 567th amino acids in the amino acid sequence of SEQ ID NO.1, which corresponds to TRAF-C domain.

11. The process according to any one of claims 8 to 10 wherein the substance that promotes or suppresses functions of the protein or antibody, wherein the protein of claim 1, or TRAF3, or the antibody of claim 7 is a substance that promotes or suppresses intracellular signal transduction via TRAF3.

12. A substance that promotes or suppresses functions of the protein of claim 1, which is obtained by the process according to any one of claims 8 to 11.

13. A medicine comprising, as an active ingredient, the substance of claim 12.

14. The medicine according to claim 13, which is used for preventing or treating diseases associated with abnormalities in intracellular signal transduction via TRAF3.

15. A diagnostic process for diseases associated with abnormalities in intracellular signal transduction via TRAF3, which comprises measuring the level of the protein of claim 1 in an organism-derived ingredient.
